# EUROPEAN PATENT SPECIFICATION

(11) **EP 0 306 968 B1**
(45) Date of publication and mention of the grant of the patent: **01.12.1993**
(21) Application number: 88114769.8
(22) Date of filing: 09.09.1988
(51) Int. Cl.: C12N 15/12, C12N 5/10, C12P 21/00

(54) **Process for producing recombinant human factor VIII:C**
Verfahren zur Herstellung des rekombinanten menschlichen Faktors VIII:C
Procédé de production de facteur VIII:C humain recombinant

(30) Priority: 10.09.1987 JP 225147/87; 08.04.1988 JP 85454/88
(43) Date of publication of application: 15.03.1989
(73) Proprietor: Juridical Foundation The Chemo-Sero-Therapeutic Research Institute, Kumamoto-shi Kumamoto-ken (JP); TEIJIN LIMITED, Osaka-shi Osaka-fu (JP)
(72) Inventor: Sugiyama, Takashi, Hino-shi Tokyo-to (JP); Masuda, Kenichi, Hachioji-shi Tokyo-to (JP); Tajima, Yoshitaka, Kumamoto-shi Kumamoto-ken (JP); Yonemura, Hiroshi, Kumamoto-shi Kumamoto-ken (JP)
(74) Representative: VOSSIUS & PARTNER

(56) References cited:
- EP-A- 0 160 457
- EP-A- 0 232 112
- WO-A-86/06101
- WO-A-87/04187
- WO-A-88/00831
- BIOCHEMISTRY, vol. 25, no. 26, 30th December 1986, pages 8343-8347, American Chemical Society, Washington, DC, US; D.L. EATON et al.: "Construction and characterization of an active factor VIII variant lacking the central one-third of the molecule"

## Description

This invention relates to a method for producing a protein having human Factor VIII:C activity. More particularly, it relates to a method for producing a recombinant human Factor VIII:C protein using genetic engineering technology.

Human Factor VIII:C is a blood plasma glycoprotein which is involved in the intrinsic pathway of blood coagulation and functions as a cofactor for Factor IXa, serine protease being capable of activating Factor X. In patients of hemophilia A which is a hereditary X chromosome-linked recessive bleeding disorder, Factor VIII:C is defective or absent in the blood. For the treatment of bleeding in hemophilia A patients caused by blood coagulation disorder, thee has clinically been used so-called Factor VIII:C replacement therapy, i.e. a method for stopping the bleeding by supplement of the Factor VIII:C, which is known as the most reasonable and reliable therapy.

For the supplement of Factor VIII:C deficient in the hemophilia A patients, there have usually been used blood plasma or cryoprecipitate obtained by freezing the blood plasma, followed by thawing of the plasma. Nowadays Factor VIII concentrates have been used for said purpose.

The source of human Factor VIII:C is human blood plasma. Commercially available Factor VIII concentrates have nowadays a content of Factor VIII:C of about 0.04 % by weight. Thus, the conventional Factor VIII concentrates usually contain various types of impurities derived from human blood plasma. While attemps have been made in the donor selection and development of technique for inactivation of viruses, there may be a danger of contamination of viruses such as those causing hepatitis or Acquired Immune Deficiency Syndrome (AIDS).

Under the circumstances, in order to obtain more reliable Factor VIII preparations at lower cost without relying on the quantitatively limited human blood plasma source and also without anxiety of the undesirable contamination of viruses contained in pooled human blood plasma, it has currently been attempted to produce Factor VIII:C using recombinant DNA technology, i.e. by cloning genes encoding human Factor VIII:C and expressing the genes in animal cells and microorganism cells.

Many attempts have been reported for the production of human Factor VIII:C by gene recombinant technology, after the gene of human Factor VIII:C was cloned, for the first time, by J. Gitschier et al (cf. Nature 312, 326 - 330, 1984), and then expressed in baby hamster kidney cells by W.I. Wood et al (cf. Nature 312, 330 - 337, 1984). The attempts are exemplified by cloning of human Factor VIII:C cDNA and its expression in mammalian cells by J.J. Toole et al (cf. Nature 312, 342 - 347, 1984), by M.A. Truett et al (cf. DNA 4 333-349, 1985), by N. Sarver et al (cf. European patent application No. 265,778), and by A. Pavirani et al (cf. Biotechnology 5, 389 - 392, 1987), respectively. In addition, the cloning of human Factor VIII:C cDNA and its expression in microorganisms by V. Ooyen et al (cf. European patent application No. 253,455) is also exemplified. However, according to these methods, the expression of the gene is transient or the gene expression levels are very low partly because the human Factor VIII:C is a glycoprotein with a very large molecular weight. Accordingly, these known methods are not satisfactory for an industrial production of human Factor VIII:C.

It is believed that the natural human Factor VIII:C has a domain structure of A1-A2-B-A3-C1-C2 in which A1, A2 and A3 have homologous amino acid sequences, and C1 and C2 have also homologous amino acid sequences (G.A. Vehar et al, Nature 312, 337 - 342, 1984). It is supposed that a part or all of the B domain is not necessary for the essential cofactor activity of human factor VIII:C. Thus, in order to increase the gene expression level in producing Factor VIII:C by recombinant DNA techniques, it has been attempted to create a molecular species which exhibits a biological activity while being modified to be as small as possible by deleting a part or all of the B domain.

### (1) Definition of natural type active human Factor VIII:C and abbreviation thereof:

Natural human Factor VIII:C is a polymorphic glycoprotein which is constituted by 2332 amino acid residues and has a molecular weight of about 300 KDa. in which the sections covering from the amino acid at the position 1 (Ala-l) up to the amimo acid at the position of 1648 (Arg-1648) are generally referred to as heavy chains while the sections from Glu-1649 up to Tyr-2332 are called light chains. Natural human Factor VIII:C is subjected to proteolytic cleavage by enzymes such as thrombin, Factor Xa and activated protein C into lower molecular fragments and the Factor VIII:C is activated or inactivated in this way.

Biologically active Factor VIII:C in plasma contains a heterogenous mixture of heavy chains ranging in length from 1648 amino acids down to 740 amino acids (of. L.-O. Andersson et al, Proc. Natl. Acad. Sci. USA 83, 2979-2983, 1986). According to Andersson's observation, the smallest biologically active Factor VIII:C consists of a heavy chain from Ala-l to Arg-740 and a light chain from 1649-Glu to Tyr-2332, wherein the heavy and the light chains are linked via a calcium-ion bridge.

In the present invention, the natural type active human Factor VIII:C is a protein which lacks a part or all of the peptide of the B domain ranging from Ser-741 to Arg-1648 of natural human Factor VIII:C, wherein the amino terminus of the light chain is covalently bonded by a peptide bond to the carboxy terminus of the heavy chain.

The natural type active human Factor VIII:C is abbreviated, for example, as follows. The natural type active human Factor VIII:C where a polypeptide fragment (H chain) ranging from Ala-l to Arg-740 is linked covalently to the amino acid of a polypeptide (L chain) ranging from Glu-1649 to Tyr-2332 is abbreviated as "740 Arg-1649 Glu Factor VIII:C". "740 Arg-1649 Glu Factor VIII:C" may be hereinafter referred to as RE.

### (2) Preparation of natural type active human Factor VIII:C molecule:

J.J. Toole et al. describe the expression of 981 Pro-1563 Asp Factor VIII:C and 759 Thr-1640 Pro Factor VIII:C in COS cells (cf. Proc. Natl. Acad. Sci. USA 83, 5939 - 5942, 1986) and in Chinese Hamster Ovary cells (CHO cells) (cf. PCT application No. WO 86/06101). R. Kaufman describes the expression of 740 Arg-1647 Gln Factor VIII:C in Chinese Hamster Ovary cells (cf. PCT application No. WO 87/04187). D.L. Eaton et al. describe the expression of 796 Gln-1563 Asp Factor VIII:C in mammalian cells (cf. Biochemistry 25, 8343 - 8347, 1986), and C. Gorman (cf. European patent application No. 260148) and by A. Pavirani et al. the expression of 116 Asp-1455 Asp Factor VIII:C in BHK-21 cells (cf. Japanese patent first publication (Kokai) No. 22195/1988). N. Sarver et al. describe the expression of 746 Ser-1561 Leu Factor VIII:C in Mouse C127 cells (cf. European patent application No. 265,778). M. Pasek et al. describe the construction of a cDNA encoding 740 Arg-1649 Glu Factor VIII:C and 744 Gln-1563 Asp Factor VIII:C and expression thereof in BMT-10 cells (R.D. Gerard et al., Mol. Cell. Biol 5, 3231 - 3240, 1985) (cf.PCT application No. WO 88/831). In microorganisms, the 866 Thr-1663 Asp Factor VIII:C gene was expressed by V. Ooyen et al. (cf. European patent application No. 253,455).

It appears that the expression levels achieved by the methods described in the above literatures are not satisfactory.

It has been attempted to express an exogenous gene by a genetic engineering technology using eukaryotic cells such as animal cells as host cells, for example, in a method employing Chinese Hamster Ovary cells deficient in dihydrofolate reductase (dhfr): dhfr(-) CHO cells (cf. G. Urlaub et al, Prot. Natl. Acad. Sci. USA 77, 4216 - 4220, 1980). The cells are eukaryotic cells which can constantly express the exogenous genes. Although there are disclosed in the PCT applications WO 86/06101 and WO 87/04187 CHO cells which can produce a specific natural type active human Factor VIII:C, the establishment of a cell line which can constantly produce 740 Arg-1649 Glu Factor VIII:C at a high production efficiency was not yet described.

The invention relates to a cell line which can constantly and continuously express exogenous genes of natural type active human Factor VIII:C at a high production efficiency, can rapidly grow in an inexpensive medium and which can substantially be propagated in a permanent manner using genetic engineering technology. The invention also relates to a transformant of animal cells (eukaryotic cells) which is prepared by transforming the animal cells with a natural type active human Factor VIII:C expression vector where the expression vector in the animal cells carries the DNA sequence encoding the natural type active human Factor VIII:C downstream of at least one promotor. An object of the invention is to provide a method for the production of recombinant human Factor VIII:C by culturing the transformant as set forth above and collecting the product accumulated in the culture medium. These and other objects and advantages of the invention will be apparent to those skilled in the art from the following description.

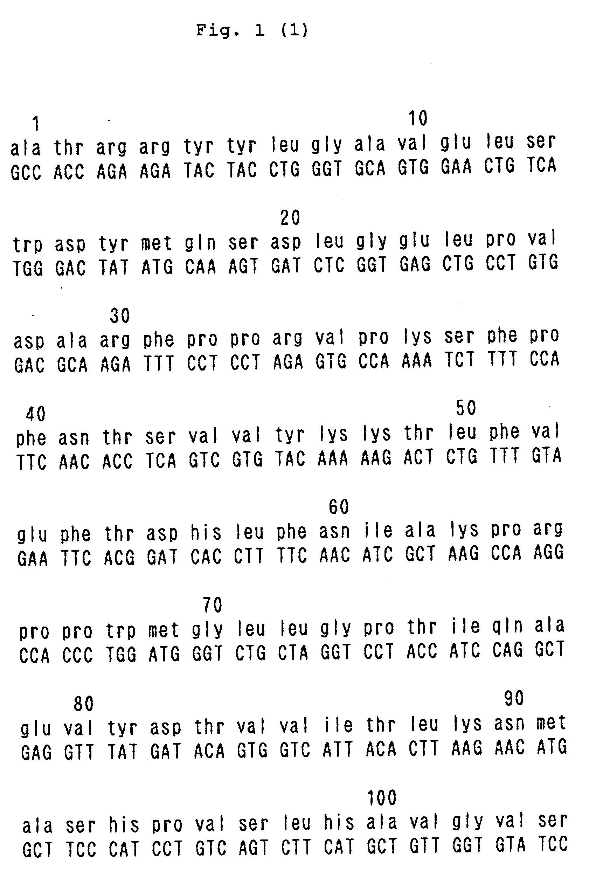

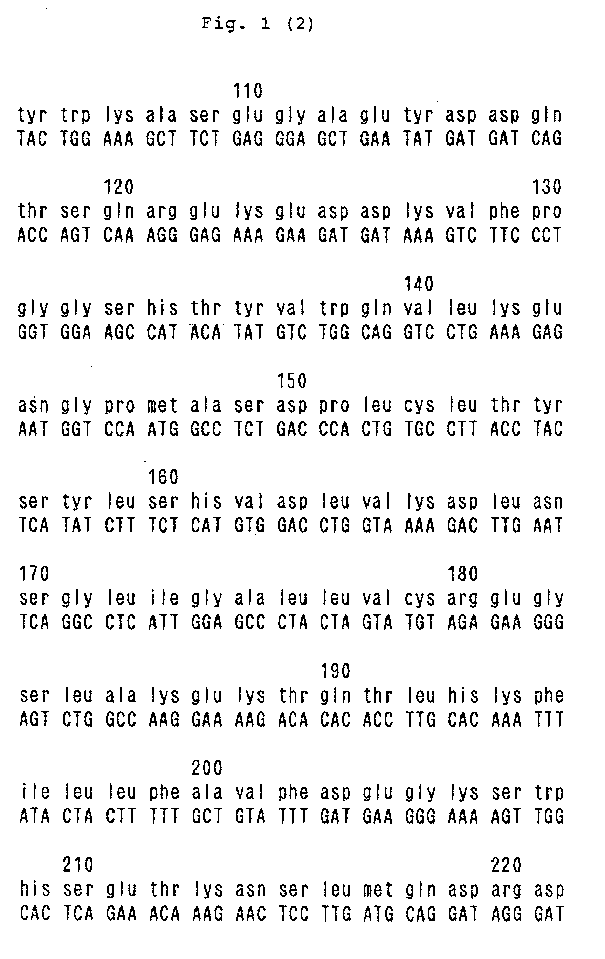

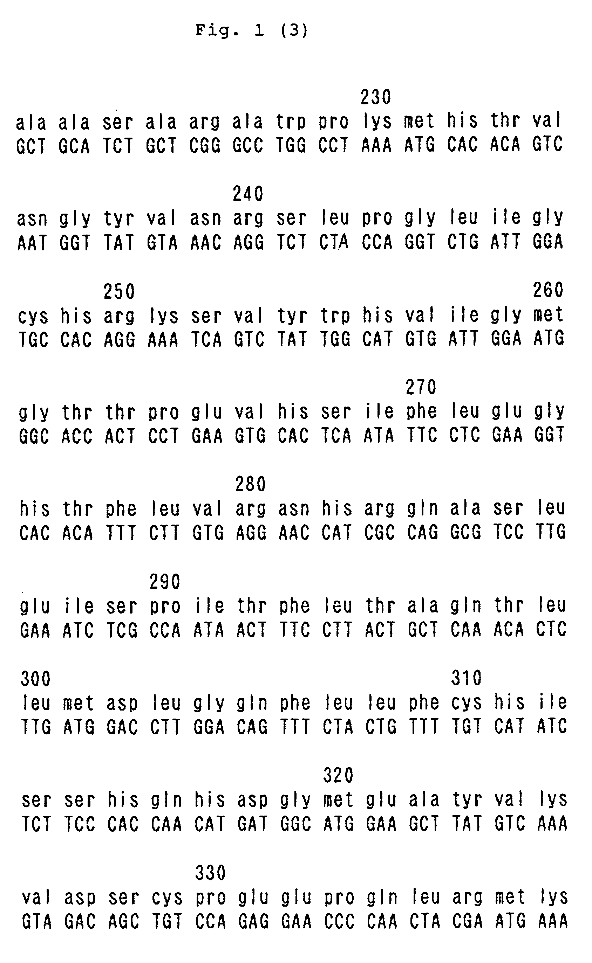

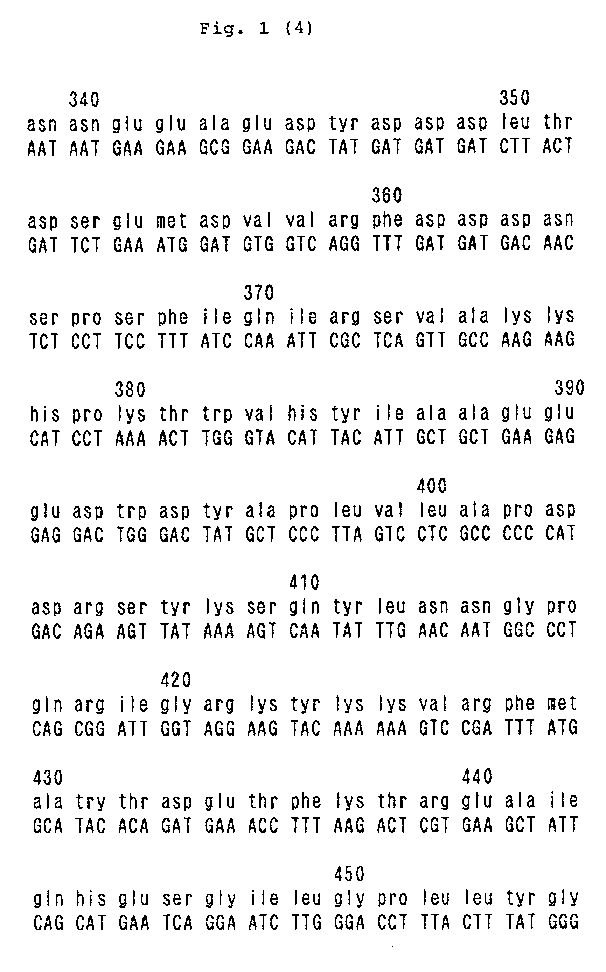

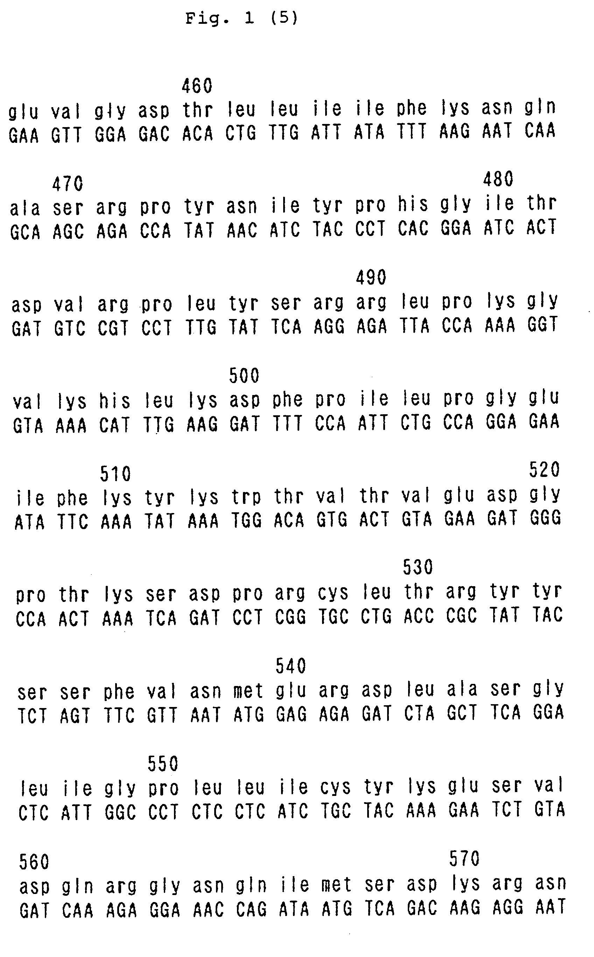

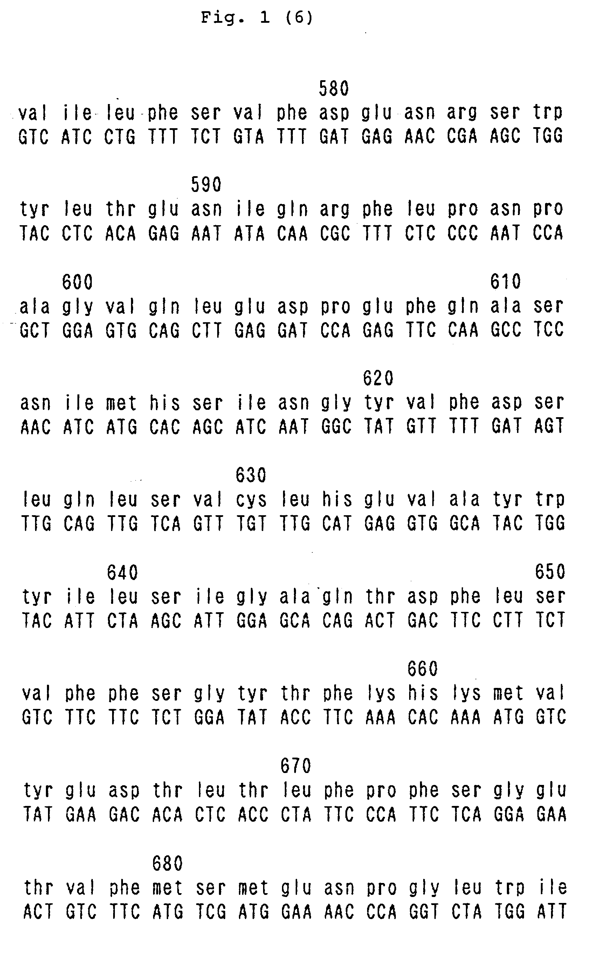

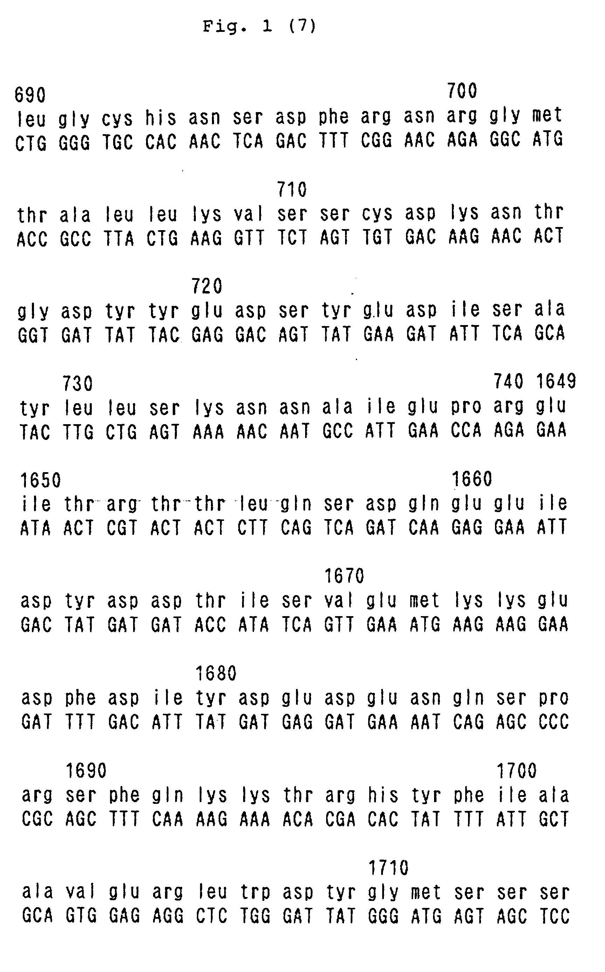

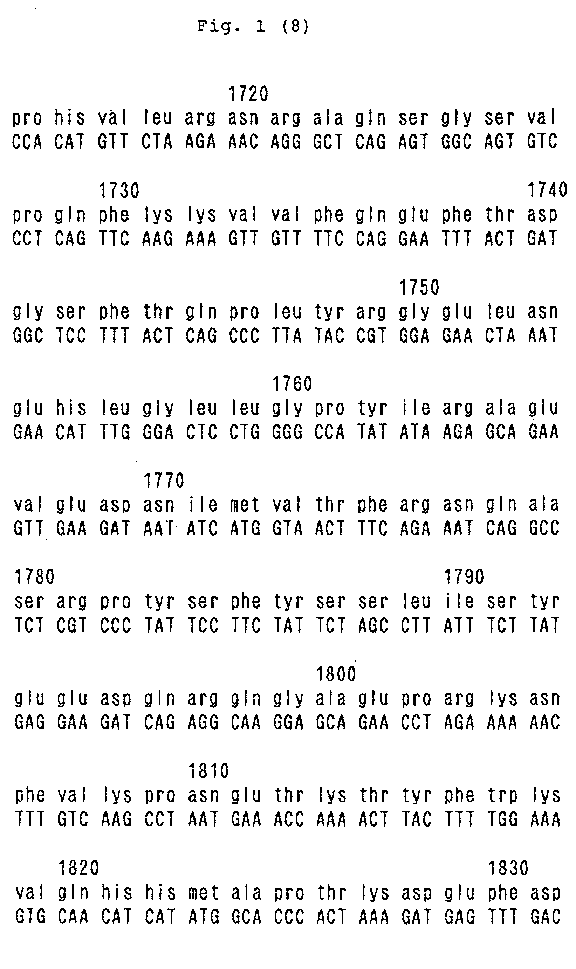

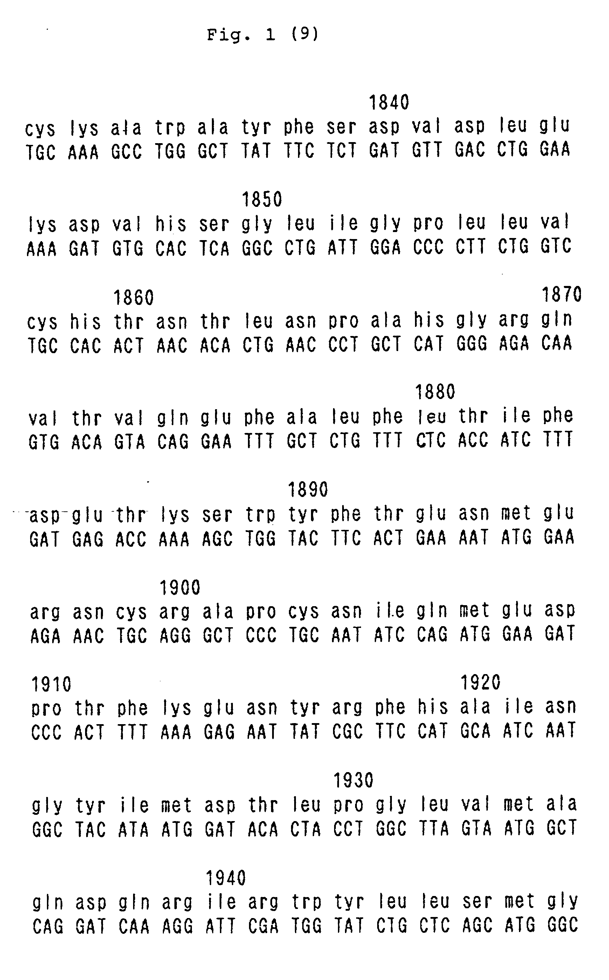

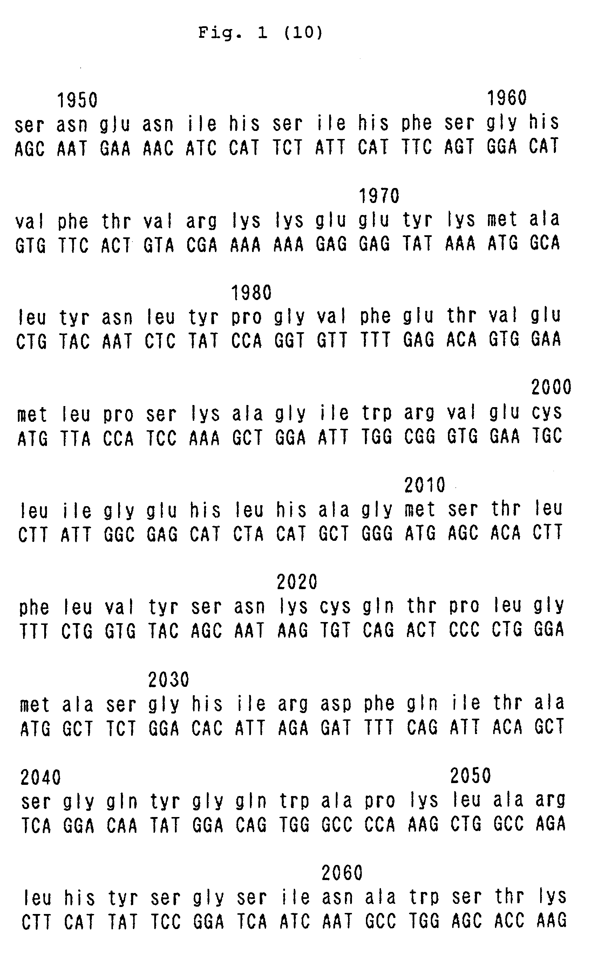

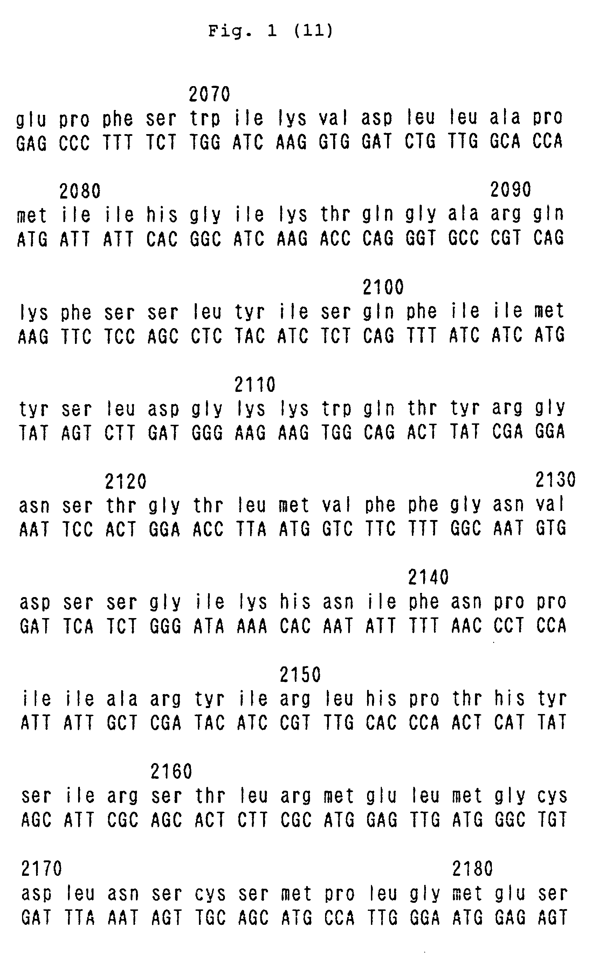

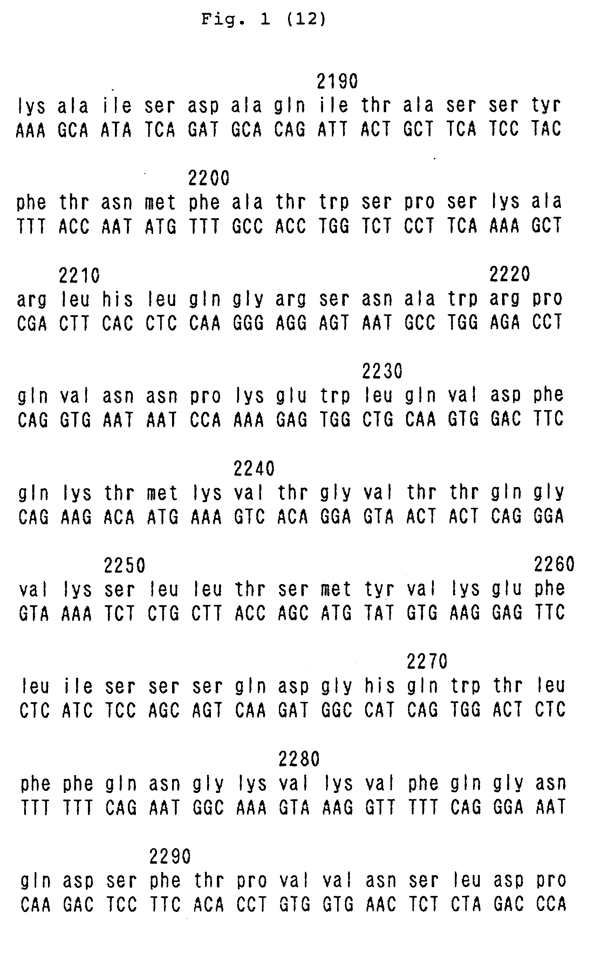

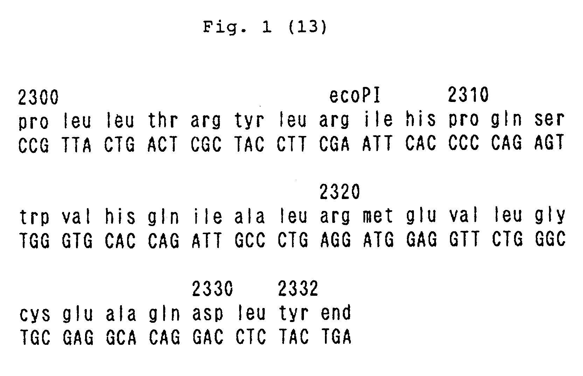

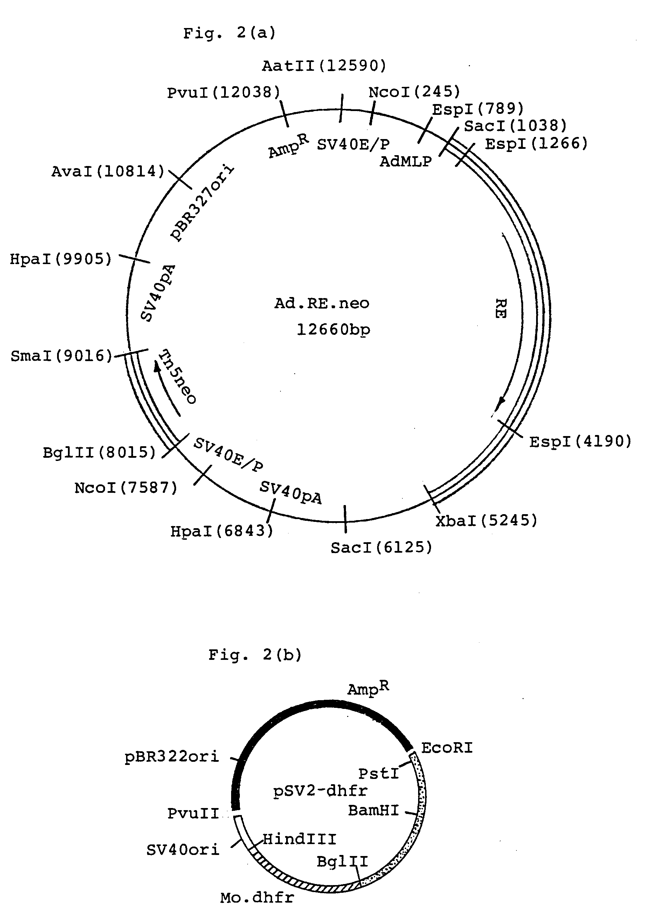

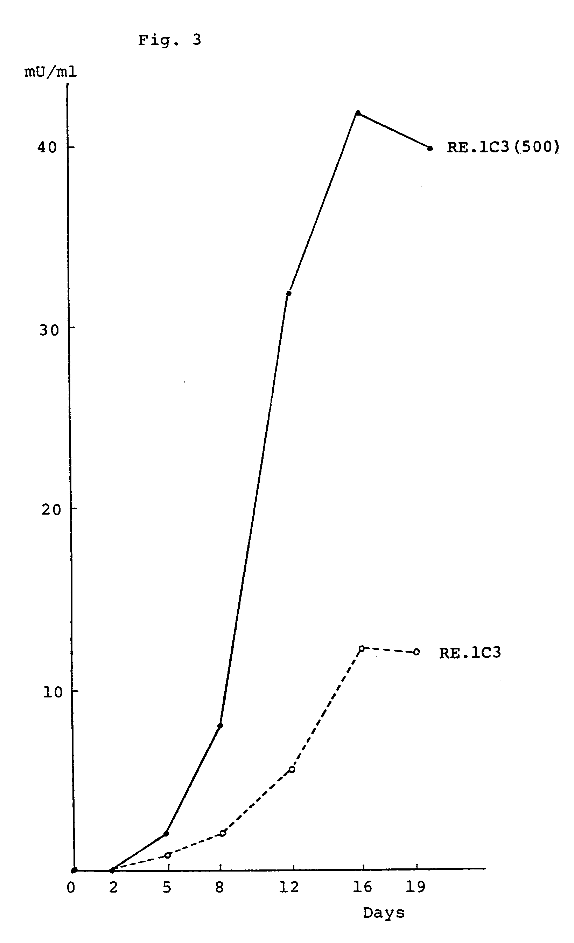

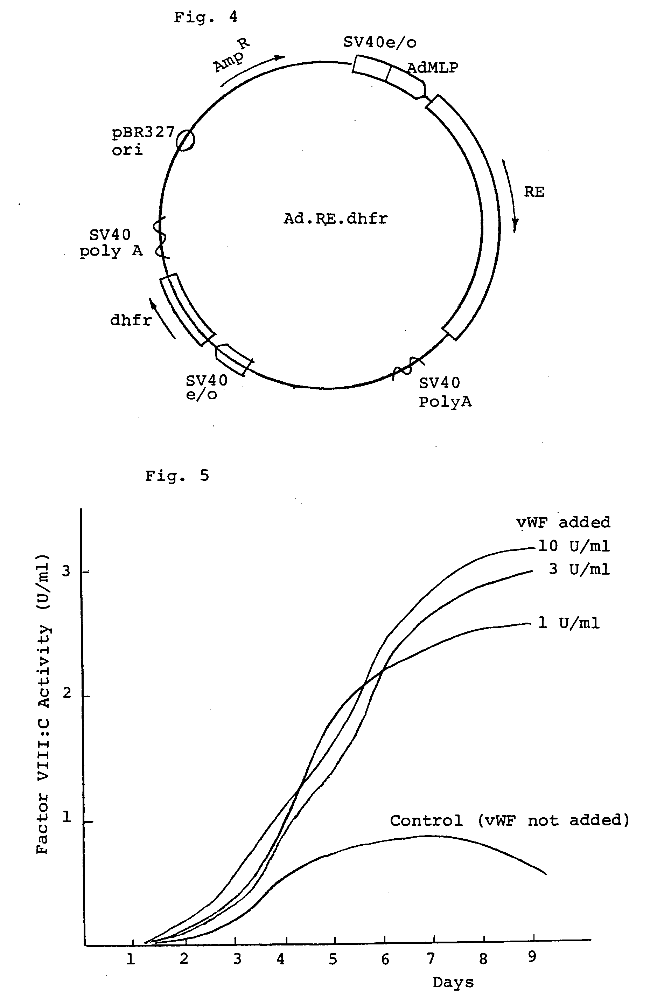

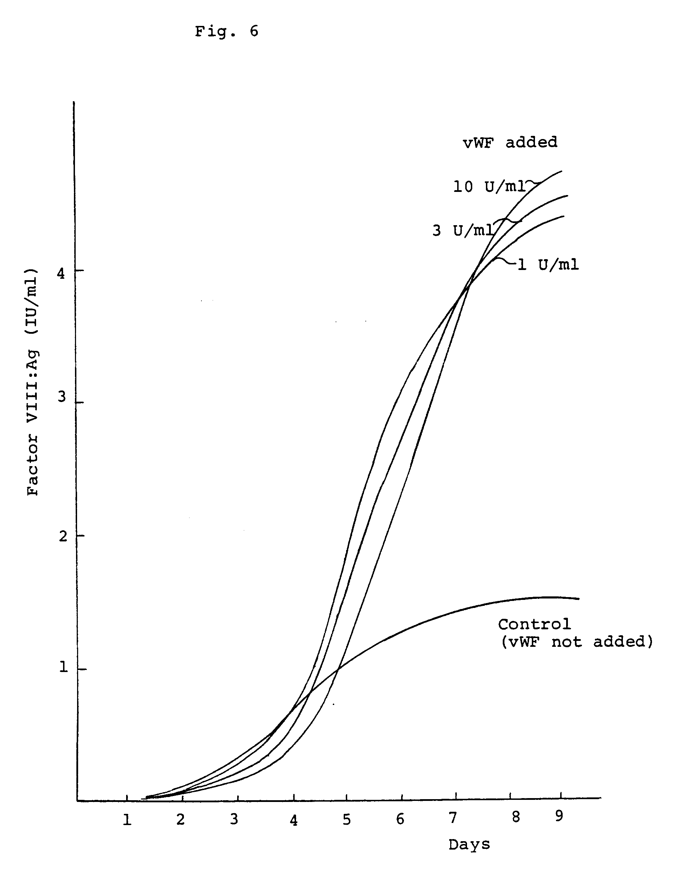

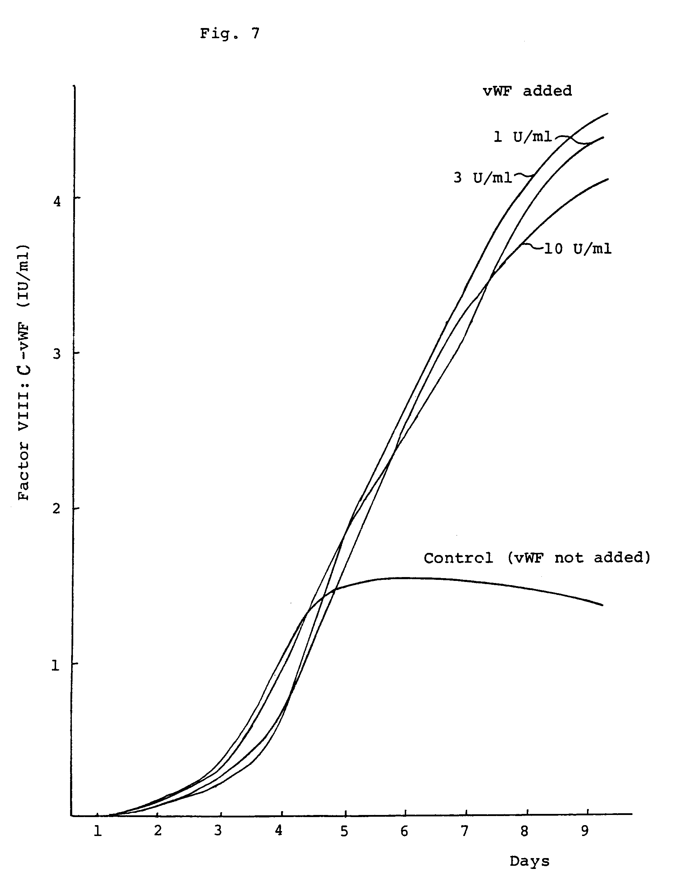

Fig. 1 shows DNA and amino acid sequences of RE (which codes for 740 Arg-1649 Glu Factor VIII:C) in plasmid Ad.RE.neo. Fig. 2 shows restriction enzyme maps of plasmid Ad.RE.neo(a) and pSV2-dhfr(b) which are used in Example 1. Fig. 3 graphically shows Factor VIII:C activity in the supernatant of culture obtained by cultivating the transformant in Example 3. Fig. 4 shows a schematic representation of plasmid Ad.RE.dhfr used in Example 5. Fig. 5 shows Factor VIII:C activity in the supernatant of culture obtained by cultivating the transformant in Example 7. Fig. 6 shows an amount of Factor VIII:C antigen in the culture medium obtained by cultivating the transformant in Example 7. Fig. 7 shows the antigen level of Factor VIII:C - von Willebrand factor complex in the culture supernant of Example 7.

The transformants used in the invention are generated by transforming animal cells with natural type active human Factor VIII:C expression vectors.

The natural type active human Factor VIII:C expression vectors carry a gene encoding a natural type active human Factor VIII:C, at least one promoter upstream thereof, optionally at least one enhancer, said promoter and enhancer being originated from animal genes and animal viruses (e.g. monkey virus or adenovirus), optionally a selectable gene for animal cells (e.g. neomycin resistance gene: transposon Tn5 or dihydrofolate reductase gene (dhfr)) and optionally an amplifiable gene (e.g. dihydrofolate reductase gene (dhfr), or adenosine deaminase gene).

In a preferred embodiment, the natural type active human Factor VIII:C expression vector contains a gene coding for natural type active human Factor VIII:C which is linked to an upstream positioned signal peptide gene (necessary for secretion) and a DNA sequence of a poly rA addition signal which is positioned downstream thereof. In this expression vector, said gene is under the control of an enhancer and a promotor which function within eukaryotic cells. Futhermore the preferred expression vector contains a neomycin-resistance gene (transposon Tn5), a replication origin for Escherichia coli and an antibiotic-resistance gene. Such an expression vector is, for example, plasmid Ad.RE.neo which is disclosed in the PCT application No. WO 88/831 wherein "RE" represents 740 Arg-1649 Glu Factor VIII:C.

RE (740 Arg-1649 Glu Factor VIII:C) has the amino acid and DNA sequence shown in Fig. 1.

The signal peptide DNA and amino acid sequence in the present invention is preferably that of natural human Factor VIII:C (cf. Nature 312, 330 - 337, 1984) but includes any ones which can precisely be cleaved between the signal peptide region and the mature protein region during the secretion process.

Any promoter which can function in eukaryotic cells can be used in the expression vector used in the present invention. Such promoters include promoters of animal virus genes and promoters of animal genes which are effective for transcription of natural type active human Factor VIII:C genes in animal cells. Suitable examples of the animal virus promoters are the SV40 early region promoter, SV40 late region promoter, adenovirus-2 major late promoter, HB virus gene promoter or the retrovirus gene promoter. Suitable examples of the animal cell gene promoter are the promoter of a thymidine kinase gene, the promotor of a metallothioneine gene or the promotor of an interferon gene. Among them, preferred promoters are the SV40 early region promoter, SV40 late region promoter, and the adenovirus-2 major late promoter. The promoters may be inducible or constitutive.

The natural type active human Factor VIII:C expression vector carries a gene conferring resistance to antibiotics or derivatives thereof (e.g. Geneticin, G418, etc.), for example, a neomycin-resistance gene, in order to make the selection of the transformed animal cells easier. Besides, linkage with a replication origin of E. coli and an antibiotic-resistance gene is useful in order to allow an easy preparation of a pure vector DNA in a large amount. The replication origin is preferably a plasmid derived from the colicine E1 plasmid, for example pBR322 and its analogous plasmids. The antibiotic-resistance genes include an ampicillin-resistance gene, a tetracycline-resistance gene, a kanamycin-resistance gene, a streptomycin-resistance gene, or a neomycin-resistance gene, and the like.

The eukaryotic host cells for the natural type active human Factor VIII:C gene expression in this invention include any animal cells which can express natural type active human Factor VIII:C gene. While a wide variety of animal cell lines are useful in expressing the genes according to the present invention, hamster cells are preferred and Chinese hamster ovary cells are more preferred. Most preferred cells are Chinese hamster ovary cells which are deficient in dihydrofolate reductase activity (DHFR), since such cells are suitable for selection and isolation of the desired transformants as well as for effective gene amplification. Particularly preferred cell lines are DHFR(-) CHO cells, DXB11, and DHFR(-) CHO cells, DG44 (cf. G. Urlaub et al., Proc. Natl. Acad. Sci. USA 77, 4216-4220, 1980), of which DG44 is more preferable in terms of stable and easy culturing.

Introduction of DNA into eukaryotic cells can be carried out by known methods, for example, by a method of F.L. Graham et al.(cf. Virology 54, 536 - 539, 1973), or a method of H. Potter et al.(cf. Proc. Natl. Acad. Sci. USA 81, 7161 - 7165, 1984). When animal cells deficient in a gene, e.g. CHO cells deficient in dihydrofolate reductase (dhfr) gene, are subjected to co-transfection (cf. Cell 16, 777 - 785, 1979) with a dhfr gene-carrying plasmid (e.g. pSV2-dhfr, cf. Mol. Cell Biol. 1, 854 - 864, 1981) and natural type active human Factor VIII:C expression vector, followed by selection in a selection medium which lacks hypoxanthine, glycine and thymidine, there can be obtained CHO cells in which dhfr genes are introduced and expressed, and natural type active human Factor VIII:C gene is possibly introduced.

When the transformation is carried out by employing eukaryotic cells in the above method, it is preferable to use a linearized plasmid transfered into the cells which is prepared by cleaving previously with appropriate restriction enzymes which do not affect the expression of the desired product, by which the desired exogenous gene can be integrated into the chromosomes of the eukaryotic cells in high efficiency.

Moreover, the animal cells into which a natural type active human Factor VIII:C gene is integrated, can express the inserted neomycin-resistance gene (transposon Tn5) together with said gene, and hence, the animal cells can easily be selected and isolated by growing the cells in a medium supplemented with a neomycin derivative (e.g. Geneticin®, G418, etc.).

The natural type active human Factor VIII:C-producing transformants can also be selected and isolated by the above-mentioned methods in combination.

Alternatively a dhfr gene is inserted in advance into above-mentioned natural type human Factor VIII:C expression vector and this composite vector is inserted into the host cells. One can effectively integrate both of the natural type active human Factor VIII:C gene and the dhfr gene into adjacent positions in the chromosomes of the host cells.

In the transformant thus prepared, amplification of the natural type active Factor VIII:C gene in the chromosomes proceeds effectively. Thus, there can be obtained a transformant which can express the natural type human Factor VIII:C gene in higher yields.

When the transformants used in the invention containing an amplifiable gene (e.g. dhfr gene, adenosine deaminase gene, etc.) are cultured under an appropriate condition suitable for gene amplification (in case of dhfr gene, the amplification is carried out in the presence of methotrexate in a concentration of 1 - 10,000 nM), the natural type active human Factor VIII:C gene carried by the transformants as well as the dhfr gene can be amplified. As a result, the transformant produces a large amount of desired protein due to the increased gene dosage, and hence, there can be produced the desired recombinant human Factor VIII:C in the culture medium in a large amount.

The transformants expressing the natural type active human Factor VIII:C gene can also be cultivated in a non-serum medium like in the conventional serum-containing medium to give the desired recombinant human Factor VIII:C. In addition, the transformants can also be cultivated in a suspension with a carrier or without a carrier.

The cultivation of the transformants may preferably be carried out by adding a substance for increasing the amount of the desired recombinant human Factor VIII:C accumulated in the medium. Such substances include preferably various types of proteins, such as albumin, von Willebrand factor and further include polyethylene glycol, sodium selenite, protease inhibitors such as ε-aminocaproic acid, PMSF (phenylmethanesulfonyl fluoride) and aprotinin, or cyclodextrins.

The recombinant human Factor VIII:C produced according to the present invention, i.e., the expression product of natural type active human Factor VIII:C gene, is synthesized as a single chain in the transformant. Some population of such expression products may be secreted into the culture medium in the form of a single chain. Another population of the products may be secreted in the form of plural-chains resulting from proteolytic cleavage in the transformant. In the case of 740 Arg-1649 Glu Factor VIII:C, the proteolytic cleavage may predominantly occur at the peptide bond between Arg-740 and Glu-1649, in which the two chains are linked to each other via a calcium-ion bridge. The resulting two chains-form active Factor VIII:C is considered to have the same structure as the active Factor VIII:C molecular species in the human blood plasma as discovered by L.-O. Andersson et al.(Proc. Natl. Acad. Sci. USA 83, 2979-2983, 1986).

The degree of the proteolytic processing in the transformant can be controlled through optimizing the conditions for the cultivation.

The recombinant Factor VIII:C protein produced and accumulated in the culture medium can be purified by removing the cells from the culture medium, concentrating the medium and subjecting the supernatant thereof to a combination of a variety of conventional purification methods and strategies.

The conventional purification methods include a method utilizing a difference in solubility (e.g. salting-out or precipitation in solvent, a method utilizing a difference in molecular weight (e.g. dialysis, ultrafiltration, gel filtration or SDS-polyacrylamide gel electrophoresis , a method utilizing a difference in electric charge (e.g. ion exchange chromatography), a method utilizing specific affinity (e.g. affinity chromatography ), a method utilizing a difference in hydrophobicity (e.g. reverse phase high performance liquid chromatography ), or a method utilizing a difference in the isoelectric point (e.g. isoelectric electrophoresis).

While the recombinant Factor VIII:C produced by this invention may be administered either in a single-chain form or in a two-chain form as they are produced, it should also be understood that it is within the scope of this invention to administer such recombinant Factor VIII:C after subjecting it to proteolytic cleavage in vitro. For example, the single-chain recombinant human Factor VIII:C as produced can be cleaved in vitro into the appropriate two-chain form followed by linking the chains via a calcium or another alkaline earth metal bridge before, during or after purification. The recombinant human Factor VIII:C of this invention may be formulated into pharmaceutically acceptable compositions using conventional methods.

The recombinant human Factor VIII:C-containing solution obtained by the present invention may optionally be lyophilized to give a powdery product. The lyophilization may be carried out in the presence of a stabilizer such as sorbitol, mannitol, dextrose, maltose, glycerol or human serum albumin (HSA) or of a combination thereof.

The transformants of animal cells of the present invention can constantly and continuously produce the desired recombinant human Factor VIII:C in high yield and can grow rapidly in an inexpensive medium, and can substantially propagate in a permanent manner. Thus, according to the present invention, the desired recombinant human Factor VIII:C can be produced in an industrial scale. Particularly, the present invention has made it possible on a commercial scale to produce a recombinant Factor VIII:C which is considered to correspond to the smallest species of active and intact Factor VIII:C molecules in the human blood plasma. The recombinant human Factor VIII:C produced by the present invention is useful for the treatment of bleeding in hemophilia A patients who are deficient in Factor VIII:C.

The present invention is illustrated by the following Examples but should not be construed to be limited thereto.

The transformant E. coli HB101(RE) prepared by transforming with an expression vector plasmid Ad.RE.neo used in Examples has been deposited as ATCC 53517 by Biogen Inc.

### Example 1

In a FALCON® bottle (culture flask) (cultivation surface area: 150 cm²), DHFR(-) CHO cells DG44 (cf. G. Urlaub et al, Proc. Natl. Acad. Sci. USA 77, 4216 - 4220, 1980, and others) were cultivated in Ham's F12 medium containing 10 % fetal bovine serum at 37°C overnight. After the cultivation, the cells were floated with trypsin and plasmid Ad.RE.neo which is a natural type active human Factor VIII:C expression vector (20 µg per 3 x 10⁶ cells) and plasmid pSV2-dhfr (cf. Mol. Cell Biol. 1, 854 - 864, 1981) (10 µg per 3 x 10⁶ cells)were introduced into the cells by an electrophoration method by H. Potter et al. (cf. Proc. Natl. Acad. Sci. USA 81, 7161 - 7165, 1984). The mixture was allowed to stand at room temperature for 5 minutes and thereafter it was cultivated in Ham's F12 medium containing 10 % fetal bovine serum for 2 days. After replacing the medium by Ham's F12 medium for selection of DHFR(+) cells containing 10 % dialyzed fetal bovine serum but lacking hypoxanthine, glycine and thymidine, the cultivation was continued at 37°C, during which the culture medium was changed at intervals of 3 to 4 days. After cultivating for about two weeks, the cells becoming DHFR(+) were grown to form colonies.

The DHFR(+) transformed cells thus obtained were further cultivated in the second selection medium, i.e. in Ham's F12 medium containing 10 % fetal bovine serum and G418 (Geneticin®, manufactured by GIBCO, 400 µg/ml). After cultivating for 1 to 2 weeks, the G418-resistant cells, neo(+) transformed cells were grown to give colonies.

Cloning of the neo(+), DHFR(+) transformed cells was carried out by a known method (e.g. limited dilution method). After the cloning, the cloned cells were cultivated in Ham's F12 medium containing 10 % dialyzed fetal bovine serum and G418 (400 µg/ml) (lacking hypoxanthine, glycine and thymidine). The cloned cells were separated and cultivated to about 80 % confluency. After replacement by a fresh medium, the cultivation was continued for 48 hours, and the activity of Factor VIII:C in the culture medium was measured by a synthetic substrate method (using Coatest® Factor VIII:C, manufactured by Kabi Vitrum) and activated partial thromboplastin coagulation time (APTT). The results are shown in Table 1.

The transformant RE.1A-3 shown in Table 1 has been deposited at Fermentation Research Institute, Agency of Industrial Science and Technology, Japan, in deposit No. FERM P-9873 (as CHO-TK-0001) on February 17, 1988, which was re-deposited at the same depositary under Budapest Treaty as FERM BP-2014 on August 24, 1988.

**Table 1**

| Transformants | Activity of Factor VIII:C (mU/ml) | |
|---|---|---|
| | Synthetic substrate method | APTT |
| RE.1A-3 | 10.4 | 9.2 |
| RE.1A-5 | 8.8 | 8.4 |
| RE.1B-1 | 7.7 | 7.9 |
| RE.1B-3 | 10.0 | 9.9 |
| RE.1C-2 | 9.7 | 9.8 |
| RE.1C-3 | 12.5 | - |
| RE.1C-5 | 9.6 | - |
| RE.2A-3 | 8.4 | - |
| RE.2A-4 | 7.3 | - |
| RE.2A-6 | 10.8 | - |
| RE.2C-3 | 7.2 | - |
| RE.2D-6 | 9.5 | - |
| RE.3A-2 | 7.3 | - |
| RE.3C-6 | 7.7 | - |
| RE.3D-5 | 6.1 | - |

### Example 2

The CHO cell line RE.1C-3 being capable of producing natural type human Factor VIII:C (740 Arg-1649 Glu Factor VIII:C) prepared in Example 1 was cultivated in Ham's F12 medium containing 20 nM methotrexate (MTX), 10 % dialyzed fetal bovine serum and G418 400 µg/ml but lacking hypoxanthine, glycine and thymidine. The grown cells were subcultured in sequentially increasing concentrations of MTX in the medium starting with 100nM and increasing to 500nM. The MTX-resistant clones were cultivated under the same conditions as used in Example 1, and the activity of Factor VIII:C in the culture medium was measured likewise. The results are shown in Table 2.

**Table 2**

| Transformants | Activity of Factor VIII:C (mU/ml) (measured by synthetic substrate method) | | | |
|---|---|---|---|---|
| | Original cells (RE.1C-3) | MTX 20 nM-resist. cells | MTX 100 nM-resist. cells | MTX 500 nM-resist. cells |
| RE.1C-3 | 12.5 | 20.7 | 26.4 | 42.0 |

### Example 3

The activity of Factor VIII:C in the culture medium of the CHO transformed cell line, RE.1C-3 prepared in Example 1 and of clone RE.1C-3(500) derived from the MTX (500 nm)-resistant cells in Example 2, was measured during a particular period of time.

In a culture flask (cultivation surface area: 150 cm²), CHO cells RE.1C-3 and CHO cells RE.1C-3(500) (each 10⁵ cells) were cultivated in Ham's F12 medium supplemented with 10 % fetal bovine serum (30 ml) at 37°C in a carbon dioxide incubator. From two days after the initiation of cultivation, the number of cells and the activity of Factor VIII:C in the culture medium were examined everyday. The results are shown in the accompanying Fig. 3. As is shown in Fig. 3, Factor VIII:C was accumulated in the culture medium with propagation of the cells, and even after the propagation of cells was stopped, Factor VIII:C was continuously produced. The maximum activity was 12.3 mU/ml in CHO cells RE.1C-3 and 42 mU/ml in CHO cells RE.1C-3(500).

### Example 4

In a culture flask (cultivation surface area: 25 cm²), DHFR(-) CHO cells DG44 and DHFR(-) CHO cells DXB11 were cultivated in MEM alpha medium containing the nucleosides (manufactured by GIBCO) supplemented with 10 % fetal bovine serum at 37°C overnight. After the cultivation, Ad.RE.neo (10µg) linearized by digestion with Pvu I and pSV2-dhfr (0.5 µg) linearized by digestion with Pvu I were introduced into the resulting cells using the DNA-calcium phosphate co-precipitation method of F.L. Graham et al. (cf. Virology, 52, 456, 1973).

After the gene introduction, the medium was replaced by a fresh one, and the cells were incubated at 37°C overnight. Thereafter, the medium was replaced by a medium for selecting DHFR(+) neo(+) cell line, i.e. MEM alpha medium supplemented with 10 % dialized fetal bovine serum and G418 (400 µg/ml) but lacking nucleosides (manufactured by GIBCO), and the cultivation was continued at 37°C. After cultivation for about 2 weeks, DHFR(+) neo(+) cells were grown to form colonies.

The DHFR(+), neo(+) transformed CHO cells DG44 and CHO cells DXB11 thus isolated were cultivated in MEM alpha medium supplemented with 20 nM MTX, 10 % dialyzed fetal bovine serum and G418 (400 µg/ml) but lacking the nucleosides. The grown cells were continuously subjected to subculturing in stepwise increasing concentrations of MTX in the medium of 100nM and 500nM. The cells were grown to about 80 % confluency. The medium was replaced by a fresh one, and the cultivation was continued for 3 days, and thereafter, the activity of Factor VIII:C in the culture medium was measured by a synthetic substrate method. The results are shown in Table 3.

**Table 3**

| Host cells | Activity of Factor VIII:C (mU/ml) (measured by synthetic substrate method) | | | |
|---|---|---|---|---|
| | Original transformant | MTX 20 nM-resist. cells | MTX 100 nM-resist. cells | MTX 500 nM-resist. cells |
| CHO cells DG44 | 5.2 | 28 | 97 | 213 |
| CHO cells DXB11 | 0.4 | 1.9 | 30 | 105 |

The DHFR(+), neo(+) CHO cells DG44 (MTX 500 nM-resistant) were cloned to give the CHO cells clones shown in Table 4 which can produce recombinant Factor VIII:C (740 Arg-1649 Gln Factor VIII:C) in high levels.

**Table 4**

| Clones | Activity of Factor VIII:C (U/ml) (measured by synthetic substrate method) |
|---|---|
| RE-KSG-1H | 0.64 |
| RE-KSG-2D | 1.06 |
| RE-KSG-3C | 0.96 |
| RE-KSG-4C | 0.94 |
| RE-KSG-7A | 0.78 |

### Example 5

We isolated a fragment of the dihydrofolate reductase gene of dhfr-expressing plasmid pSV2-dhfr as used in Example 1 by digesting with the restriction enzymes Pvu II and EcoR I and then filling the 5'AATT protruding end with T₄ DNA polymerase in order to obtain blunt ended DNA. A fragment carrying the dhfr gene was isolated by electrophoresis in a 0.7 % agarose gel.

Separately, the natural type active human Factor VIII:C-expressing plasmid Ad.RE.neo was digested with the restriction enzyme Sma I and then the above dhfr gene fragment was inserted with T₄ DNA ligase. The resulting plasmid Ad.RE.dhfr is a vector being capable of expressing both the natural type human Factor VIII:C gene and the dihydrofolate reductase gene.

The plasmid Ad.RE.dhfr was linearized by digesting with the restriction enzyme Pvu I, and the resulting linear DNA (10 µg) was mixed with a carrier DNA (calf thymus DNA, 10 µg) in 0.25 M CaCl₂ (100 µl), and in HEPES buffered Saline (100 µl) was added dropwise. The DNA-calcium phosphate complex precipitate thus prepared was added to DHFR(-) CHO cells DG44 that were previously subcultured in the same manner as described in Example 1.

The cells were cultivated for 15 hours and after replacing the medium by MEM alpha medium containing no ribonuleosides and deoxyribonucleosides (manufactured by GIBCO) supplemented with 10 % dialyzed fetal bovine serum (hereinafter, referred to as "a medium for selecting dhfr-expressing cells"), the cultivation was continued to give the DHFR(+)-transformed CHO cell RED44. After cultivating for 12 days, the activity of Factor VIII:C in the culture medium was measured by a synthetic substrate method. As a result, it was expressed in an amount of 10 mU/ml.

Subsequently, the RED44 cells were cloned by a limited dilution method.

After the cloning the cells were plated in a 24 wells culture plate (manufactured by Nunk) and were cultivated in a medium for selecting dhfr-expressing cells (1 ml). We grew each cloned cell to confluency and then the activity of Factor VIII:C in the culture medium was measured by a synthetic substrate method. The results are shown in Table 5.

**Table 5**

| Clones | Activity of Factor VIII:C (U/ml) (measured by synthetic substrate method) |
|---|---|
| A7 | 126 |
| D2 | 117 |
| D7 | 123 |
| D12 | 98 |
| 3C8 | 85 |
| 2E8 | 58 |

### Example 6

Four clones of CHO cell RED44 being capable of producing recombinant Factor VIII:C isolated in Example 5 (shown in Table 5) were transferred into a FALCON® 6 wells culture plate and cultivated in the medium for selecting DHFR-expressing cells which contained 10 nM methotrexate (MTX). The cells were cultured in sequentially increasing concentrattions of MTX in the medium starting with 50 nM and then with 100 nM. The cells were grown to 95 % confluency, and three or four days after changing the medium, the activity of Factor VIII:C in the culture medium was measured likewise. The results are shown in Table 6.

**Table 6**

| Clones | Activity of Factor VIII:C (mU/ml) (measured by synthetic substrate method) | | |
|---|---|---|---|
| | MTX concentration: 10 nM | 50 nM | 100 nM |
| A7 | 490 | 800 | 1080 |
| D2 | 120 | 670 | 880 |
| D7 | 180 | 690 | 842 |
| D12 | 420 | 704 | 1100 |

Among the CHO cells being capable of producing natural type human Factor VIII:C, the CHO cell RED44. A7 shown in Table 6 was deposited at Fermentation Research Institute, Agency of Industrial Science and Technology, Japan, in deposit No. FERM P-9935 (as CHO RED44.A7) on March 11, 1988, which was re-deposited at the same depositary as FERM BP-2016 on August 25, 1988.

### Example 7

The MTX-resistant CHO cells RED44.A7 being capable of producing recombinant Factor VIII:C cloned in Example 5 were transferred into a FALCON® 6 well culture plate (#3046) and cultivated in the medium for selecting DHFR-expressing cells (4 ml) in the presence of 50 nM methotrexate, to which purified human von Willebrand factor was added in a concentration of 0, 1, 3, and 10 U/ml, respectively. While continuing the cultivation, a sample of the culture medium was intermittently taken out and tested.

The activity of Factor VIII:C in the samples was then measured by a synthetic substrate method, and further, the amount of Factor VIII:C antigen (Factor VIII:C Ag) was measured by ELISA using anti-Factor VIII:C monoclonal antibodies. Moreover, antigen levels of the Factor VIII:C - von Willebrand factor complex were measured by ELISA using a monoclonal anti-human Factor VIII:C as the first antibody and a rabbit polyclonal anti-human von Willebrand factor antibody (cf. Acta Heamatol. Jpn. 50, 1681 - 1688, 1987). The results of activity of Factor VIII:C measured by the synthetic substrate method are shown in the accompanying Fig. 5. The results of the amount of Factor VIII:C antigen measured by ELISA and the antigen level of Factor VIII:C - von Willebrand factor complex measured by ELISA are shown in Fig. 6 and Fig. 7, respectively.

### Example 8

The MTX-resistant CHO cells RED44.A7 being capable of producing natural type human Factor VIII:C prepared in Example 5 were transferred into a FALCON® 6 wells tissue culture plate and cultivated in the medium for selecting DHFR-expressing cells (3 ml) which contained 50 nM methotrexate for 24 hours. The medium was then removed and the cells were incubated in commercially available non-serum medium (ASF medium 104, manufactured by Ajinomoto Co., Inc., 3 ml). While continuing the cultivation, samples of the culture medium were intermittently taken out and tested. The activity of Factor VIII:C in the samples was then measured by a synthetic substrate method. As a control experiment, a conventional serum medium (MEM alpha medium containing 10 % fetal calf serum (FCS) was used. The above assays were made at 3, 5 and 7 days after changing the medium. The results are shown in Table 7.

**Table 7**

| Medium | Activity of Factor VIII:C (mU/ml) (synthetic substrate method) | | |
|---|---|---|---|
| | After 3 days | 5 days | 7 days |
| ASF Medium 104 | 550 | 820 | 570 |
| 10 % FCS-containing MEM alpha medium | 50 | 240 | 270 |

In addition, in the same manner as described above except that various additives such as polyethylene glycol (PEG) 20,000, sodium selenite, ε-aminocaproic acid, phenylmethanesulfonyl fluoride (PSMF), human von Willebrand factor (h-vWF), bovine serum albumin (BSA), aprotinin or α-cyclodextrin were added to the non-serum medium and that the concentration of methotrexate was 100 nM, the MTX-resistant CHO cell RED44.A7 was cultivated, and the activity of Factor VIII:C in the culture medium was measured by a synthetic substrate method during a particular period of time. The results are shown in Table 8 to Table 11.

Factor VIII:C antigen was examined in a similar experiment using aprotinin with the result that the expression level after the addition of 100 to 10000 KIU/ml is two or three times higher than the control without addition of aprotinin.

**Table 8**

| (Effects of each additive to non-serum medium) | | | |
|---|---|---|---|
| Additives | Activity of Factor VIII:C (mU/ml) (synthetic substrate method) | | |
| | After 2 days | 4 days | 9 days |
| PEG 20,000 (0.1 %) | 9 | 136 | 705 |
| Sodium selenite (13 µg/l) | 18 | 95 | 664 |
| PEG + sodium selenite | 33 | 123 | 830 |
| α-Cyclodextrin (0.1 %) | 10 | 150 | 953 |
| Non-additive | 15 | 104 | 498 |

**Table 9**

| (Effects of addition of ε-aminocaproic acid to non-serum medium) | | | |
|---|---|---|---|
| Additives | Activity of Factor VIII:C (mU/ml) (synthetic substrate method) | | |
| | After 5 days | 7 days | 10 days |
| 1 mM ε-aminocaproic acid | 338 | 484 | 620 |
| Non-additive | 230 | 351 | 125 |

**Table 10**

| (Effects of addition of PMSF, h-vWF to non-serum medium) | | | |
|---|---|---|---|
| Additives | Activity of Factor VIII:C (mU/ml) (synthetic substrate method) | | |
| | After 2 days | 4 days | 7 days |
| 0.1 mM PMSF | 103 | 381 | 784 |
| 2U h-vWF | 332 | 913 | 2910 |
| Non-additive | 148 | 290 | 747 |

**Table 11**

| (Effects of addition of BSA to non-serum medium, cultivation for 2 days) | |
|---|---|
| Concentration of BSA | Activity of Factor VIII:C (mU/ml) (synthetic substrate method) (µg/ml) |
| 0 | 62 |
| 10 | 50 |
| 50 | 83 |
| 100 | 54 |
| 1000 | 249 |
| 10000 | 540 |

## Claims

1. A method for producing a protein having human Factor VIII:C activity and consisting of the heavy and light chain of human Factor VIII:C, by cultivating, in a nutrition medium, transformed animal cells containing an expression vector comprising
- the DNA sequences corresponding to the heavy and light chain which are linked to a signal peptide sequence
- at least one promoter upstream thereof, and
- at least one enhancer
and recovering the protein having human Factor VIII:C activity accumulated in the nutrition medium
characterized in that
the cultivation is carried out by adding to the nutrition medium albumin, polyethylene glycol, sodium selenite, ε-aminocaproic acid, phenylmethanesulfonyl fluoride (PMSF), aprotinin or cyclodextrins.

2. A method according to claim 1, wherein the cultivation of the transformant is carried out under conditions suitable for inducing gene amplification.

3. A method according to claim 1 or 2, wherein the cultivation of the transformant is carried out in a serum-free medium.

## Patentansprüche

1. Verfahren zur Herstellung eines Proteins mit der Aktivität des menschlichen Faktors VIII:C, das aus der schweren und leichten Kette des menschlichen Faktors VIII:C besteht, durch Züchtung von transformierten tierischen Zellen in einem Nährmedium, welche einen Expressionsvektor enthalten, umfassend
die DNA-Sequenzen, die der schweren und leichten Kette entsprechen, welche zu einer Signalpeptidsequenz verknüpft sind,
mindestens einen Promotor stromaufwärts davon, und mindestens einen Enhancer,
und Gewinnung des Proteins mit der Aktivität des menschlichen Faktors VIII:C, das sich im Nährmedium angereichert hat,
dadurch gekennzeichnet, daß die Züchtung unter Zugabe von Albumin, Polyethylenglykol, Natriumselenit, ε-Aminocapronsäure, Phenylmethansulfonylfluorid (PMSF), Aprotinin oder Cyclodextrinen zum Nährmedium durchgeführt wird.

2. Verfahren nach Anspruch 1, wobei die Züchtung des Transformanten unter Bedingungen durchgeführt wird, die für die Induktion einer Genamplifikation geeignet sind.

3. Verfahren nach Anspruch 1 oder 2, wobei die Züchtung des Transformanten in einem serumfreien Medium durchgeführt wird.

## Revendications

1. Procédé de production d'une protéine présentant l'activité du facteur VIII:C humain et consistant en la chaîne lourde et en la chaîne légère du facteur VIII:C humain,par culture, dans un milieu nutritif, de cellules animales transformées contenant un vecteur d'expression comprenant :
- les séquences d'ADN correspondant à la chaîne lourde et à la chaîne légère qui sont liées à une séquence de peptide signal,
- au moins un promoteur en amont des précédentes, et
- au moins un amplificateur,
et récupération de la protéine présentant l'activité du facteur VIII:C humain accumulée dans le milieu nutritif, caractérisé en ce que la culture est réalisée en ajoutant au milieu nutritif de l'albumine, du polyéthylèneglycol, du sélénite de sodium, de l'acide ε-aminocaproïque, du fluorure de phénylméthanesulfonyle (PMSF), de l'aprotinine ou des cyclodextrines.

2. Procédé selon la revendication 1, dans lequel la culture du transformant est accomplie dans des conditions appropriées pour induire une amplification génique.

3. Procédé selon la revendication 1 ou 2, dans lequel la culture du transformant est réalisée dans un milieu exempt de sérum.
